# DEMANDE DE BREVET EUROPEEN

(11) **EP 2 622 992 A1**
(43) Date de publication de la demande: **07.08.2013**
(21) Numéro de dépôt: 13154279.7
(22) Date de dépôt: 06.02.2013
(51) Int. Cl.: A47C 7/74, A47C 21/04

(54) **Elément d'ameublement avec dispositif de circulation d'air**

(30) Priorité: 06.02.2012 FR 1251068
(71) Demandeur: Pierron, François, 57130 Jouy-Aux-Arches (FR)
(72) Inventeur: Pierron, François, 57130 Jouy-Aux-Arches (FR)
(74) Mandataire: Blot, Philippe Robert Emile

(57) **Abrégé**

Elément d'ameublement (1), destiné à recevoir au moins une personne en position assise ou couchée, comprenant une armature (2) et une couche de soutien (4) comportant une surface (8) destinée à être en contact avec ladite personne, caractérisé en ce qu'il comprend en outre un dispositif (10, 6) pour faire circuler un fluide à travers ladite surface (8).

Procédé de création d'une ambiance autour d'une personne en position assise ou couchée sur un tel élément d'ameublement (1).
Application à un lit ou à un fauteuil.

## Description

La présente invention concerne un élément d'ameublement destiné à recevoir au moins une personne en position assise ou couchée, comprenant une armature et une couche de soutien comportant une surface destinée à être en contact avec ladite personne. L'élément d'ameublement peut notamment être un lit ou un fauteuil.

Lorsqu'une personne utilise un tel élément d'ameublement, elle peut souhaiter, pour améliorer son confort, contrôler l'ambiance dans laquelle elle se trouve.

Il est ainsi connu, pour que la personne puisse se réchauffer, d'utiliser, en appoint de l'élément d'ameublement, une couverture chauffante pourvue de résistances électriques et disposée sur la personne.

Il est également connu de climatiser la pièce dans laquelle se trouve l'élément d'ameublement en question. Toutefois, la climatisation n'est parfois pas uniforme dans la pièce, et les conditions, notamment de température, ressenties par la personne allongée dans un lit ou assise dans un fauteuil, sont souvent très différentes de la consigne donnée aux appareils de chauffage ou de climatisation de la pièce. Ces différences peuvent s'expliquer, selon le cas, par des difficultés à climatiser la pièce, liées par exemple à l'inertie thermique de la pièce et/ou des appareils de climatisation.

En outre, les couvertures d'un lit ou la structure même d'un lit ou d'un fauteuil constituent une isolation totale ou partielle entre la personne et l'atmosphère de la pièce, expliquant par exemple que la personne ait trop chaud, alors que la pièce est climatisée à une température relativement basse.

Par ailleurs, la chaleur dégagée par la personne elle-même s'avère parfois un problème, notamment si l'élément d'ameublement est très isolant, alors que la personne a trop chaud. A contrario, la personne ressent parfois que le fauteuil ou le lit froid dans lequel elle vient de prendre place met, du fait de sa structure isolante, un temps relativement long à se réchauffer à son contact, par exemple si la pièce est peu chauffée.

Il est également connu d'humidifier ou de parfumer l'atmosphère d'une pièce, soit pour des raisons d'agrément, soit pour faciliter la respiration d'une personne. Toutefois, à nouveau, les mêmes difficultés existent parfois pour humidifier ou parfumer l'air d'une pièce de manière efficace, par exemple du fait du volume de la pièce ou de courants d'air dans la pièce.

Pour résoudre ces problèmes, la personne est généralement tentée, au moins dans un premier temps, sinon en permanence, d'augmenter l'intensité de la climatisation pour obtenir rapidement un résultat, ce qui conduit à des problèmes de régulation de l'ambiance et à des gaspillages, selon le cas, d'énergie, d'eau, et/ou de parfum.

Un but de l'invention est de résoudre ces problèmes, en proposant un élément d'ameublement permettant de contrôler de manière plus efficace l'ambiance autour de la personne utilisant un élément d'ameublement du type précité.

A cet effet, l'invention a pour objet un élément d'ameublement destiné à recevoir au moins une personne en position assise ou couchée, comprenant une armature et une couche de soutien comportant une surface destinée à être en contact avec ladite personne, caractérisée en ce qu'il comprend en outre un dispositif pour faire circuler un fluide à travers ladite surface.

Par « faire circuler un fluide à travers ladite surface », on entend que le fluide peut circuler de l'intérieur de l'élément d'ameublement vers l'extérieur, ou bien l'inverse, ou bien encore dans les deux sens dans des zones différentes de la surface destinée à être en contact avec ladite personne.

Par « contact avec la personne », on entend un contact direct, ou bien indirect, c'est-à-dire qu'il peut, par exemple, y avoir un drap ou tout élément poreux vis-à-vis de l'air entre la surface de la couche de soutien et la personne.

Par « parfum », on entend aussi, le cas échéant une huile essentielle.

Selon des modes particuliers de réalisation, l'élément d'ameublement peut comprendre l'une ou plusieurs des caractéristiques suivantes, prise(s) isolément ou selon toutes les combinaisons techniquement possibles :
- le fluide est de l'air ou de l'air enrichi par exemple en oxygène, ou de l'air purifié ;
- le dispositif pour faire circuler le fluide à travers ladite surface comprend un système distributeur et collecteur de fluide comportant une pluralité de canaux pour réaliser, sélectivement, une injection du fluide vers l'extérieur de la couche de soutien et/ou une aspiration du fluide vers l'intérieur de la couche de soutien, dans une ou plusieurs zones prédéterminées de ladite couche de soutien ;
- le système distributeur et collecteur de fluide comprend une canalisation principale et, dans chaque zone prédéterminée, une ou plusieurs cavités s'étendant dans la couche de soutien sous la surface pour distribuer l'air injecté et/ou aspiré ;
- le système distributeur et collecteur de fluide comprend, dans chaque zone prédéterminée, un ou plusieurs serpentins situés dans couche de soutien sous la surface, pour faire circuler le fluide de et/ou vers la surface, les serpentins comprenant une pluralité d'orifices répartis le long de chaque serpentin ;
- les orifices sont réglables individuellement entre une position ouverte et une position fermée et en ce que le dispositif pour faire circuler le fluide comprend des moyens pour faire circuler un liquide de température contrôlée, sélectivement dans au moins un des serpentins ;
- l'élément d'ameublement comprend en outre un ensemble frigorifique pour refroidir, préalablement à son injection, le fluide destiné à être injecté dans une ou plusieurs zones à refroidir prises parmi lesdites une ou plusieurs zones prédéterminées ;
- l'élément d'ameublement comprend en outre un ensemble de chauffage pour chauffer, préalablement à son injection, le fluide destiné à être injecté dans une ou plusieurs zones à chauffer prises parmi lesdites une ou plusieurs zones prédéterminées ;
- l'élément d'ameublement comprend en outre un appareil de distribution de parfum, ou d'une substance chimique, et/ou d'humidité, dans le fluide destiné à être injecté dans une ou plusieurs zones à parfumer et/ou à humidifier prises parmi lesdites une ou plusieurs zones prédéterminées ;
- la couche de soutien comprend un coussin ou un matelas et une housse, la surface destinée à être en contact avec ladite personne appartenant à ladite housse, le système distributeur et collecteur de fluide se situant soit dans la housse, soit dans ledit coussin ou matelas, soit entre la housse et ledit coussin ou matelas ;
- la surface destinée à être en contact avec ladite personne comprend un revêtement perforé, respirant et nettoyable.

L'invention concerne également un procédé de création d'une ambiance autour d'une personne en position assise ou couchée sur un élément d'ameublement tel que décrit ci-dessus, comportant les étapes suivantes :
- sélection de zones à traiter ;
- pour chaque zone à traiter sélectionnée, choix d'un ou plusieurs traitements pris parmi l'injection de fluide refroidi, l'injection de fluide chauffé, l'injection de fluide humidifié et/ou parfumé et l'aspiration de fluide ;
- pour chaque traitement choisi, sélection d'une intensité du traitement choisi ; et
- à l'aide dudit dispositif pour faire circuler du fluide à travers ladite surface, dans chaque zone de traitement sélectionnée, injection et/ou aspiration à travers la zone de traitement sélectionnée, selon le traitement choisi, d'un ou plusieurs débits de fluide choisi pour réaliser lesdits traitements choisis avec lesdites intensités sélectionnées.

L'invention sera mieux comprise à la lecture de la description qui va suivre, donnée uniquement à titre d'exemple, et faite en se référant aux dessins annexés, sur lesquels :
- la figure 1 est une vue schématique, de côté, d'un lit selon l'invention,
- la figure 2 est une vue de dessus du lit représenté sur la figure 1,
- la figure 3 est un schéma principe d'un sélecteur de voies contenu dans le lit des figures 1 et 2,
- la figure 4 représente une variante, en vue de côté, du lit représenté sur les figures 1 et 2,
- la figure 5 est une vue schématique, de côté, d'un fauteuil selon l'invention,
- la figure 6 est une vue de dessus des coussins du fauteuil représenté sur la figure 5,
- la figure 7 représente une variante, en vue de côté, du fauteuil représenté sur les figures 5 et 6,
- la figure 8 représente une variante, en vue de dessus, du lit représenté sur les figures 1 et 2,
- la figure 9 illustre un détail de la figure 8, en vue de côté.

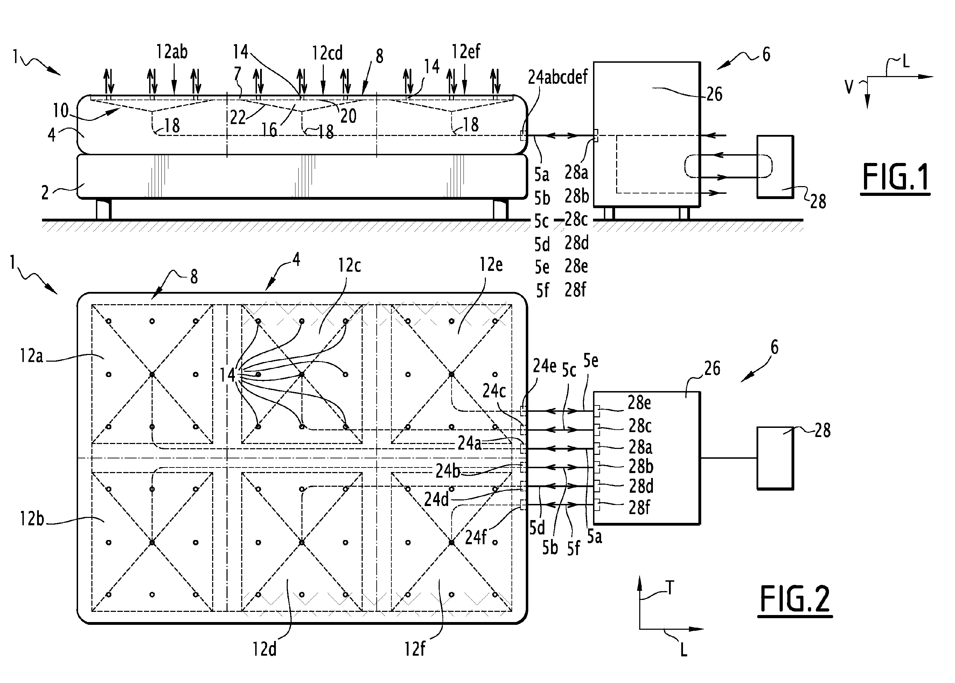

Sur les figures 1 et 2, l'élément d'ameublement selon l'invention est un lit 1, par exemple à deux places. Le lit 1 comprend une armature, ici un sommier 2, une couche de soutien, ici un matelas 4, et une installation de conditionnement d'air 6 placée à côté du matelas 4 et connectée à celui-ci par des tubes de connexion 5a, 5b, 5c, 5d, 5e, 5f. Le lit 1 ainsi qu'une partie de l'installation de conditionnement d'air 6 se trouvent dans une même pièce (non-représentée), avec deux des pièces séparées.

Le sommier 2 et le matelas 4 définissent une direction longitudinale L, horizontale et correspondant à l'orientation générale du corps des personnes (non-représentées) couchées dans le lit 1, et une direction transversale T, horizontale et perpendiculaire à la direction longitudinale L.

Le sommier 2 est un sommier classique, par exemple de forme rectangulaire.

Le matelas 4 repose sur le sommier 2. Le matelas 4 comprend une couche de contact 7 dont la surface supérieure forme une surface 8 destinée à être en contact avec les personnes couchées dans le lit 1 et un système distributeur ou collecteur de fluide 10.

Le fluide est de l'air. En variante, le fluide pourrait être de l'air enrichi, par exemple en oxygène, ou même de l'oxygène pur, par exemple pour une personne présentant des troubles respiratoires. Ce qui est mentionné ci-après avec l'air comme fluide vaut aussi pour de l'air enrichi, ou l'oxygène.

Le système distributeur ou collecteur d'air 10 du matelas 4 et l'installation de conditionnement d'air 6 forment un dispositif pour faire circuler l'air à travers la surface 8 en contact avec la personne couchée dans le lit 1.

Selon un mode de réalisation particulier, le matelas 4 se divise en six zones prédéterminées 12a, 12b, 12c, 12d, 12e, 12f pour la circulation d'air à travers la surface 8.

La première zone prédéterminée 12a et la seconde zone prédéterminée 12b occupent environ le tiers supérieur du matelas 4 selon la direction longitudinale L et sont destinées à se situer au niveau de la tête des personnes allongées sur le lit 1. La première zone prédéterminée 12a et la seconde zone prédéterminée 12b se succèdent selon une direction transversale T du matelas 4. Chacune s'étend sur environ une moitié du matelas 4 selon la direction transversale T.

La troisième zone prédéterminée 12c et la quatrième zone prédéterminée 12d occupent le tiers médian du matelas 4 selon la direction longitudinale L. Elles sont destinées à se situer au niveau du tronc des personnes allongées sur le lit 1.

La cinquième zone prédéterminée 12e et la sixième zone prédéterminée 12f occupent le tiers inférieur du matelas 4 selon la direction longitudinale L. Elles sont destinées à se situer au niveau des jambes des personnes allongées sur le lit 1.

Les zones prédéterminées 12a, 12b, 12c, 12d, 12e, 12f du matelas 4 ont des structures similaires, aussi, sur les figures, le système distributeur et collecteur d'air 10 est référencé seulement dans la troisième zone prédéterminée 12c.

Le système distributeur et collecteur d'air 10 du matelas 4 comprend, pour chaque zone prédéterminée 12a, 12b, 12c, 12d, 12e, 12f des canaux 14 situés dans la couche de contact 7, une cavité 16 et une canalisation principale 18.

Les canaux 14 sont sensiblement orientés selon la direction verticale V. Vers le haut, ils débouchent sous la forme d'orifices sur la surface 8 destinée à être en contact avec les personnes. Les orifices sont barrés d'une grille souple (non-représentée) s'étendant dans la continuité de la surface 8 destinée à être en contact avec la personne. Vers le bas, les canaux 14 débouchent dans la cavité 16 située dans le matelas 4.

La cavité 16 s'étend sous la couche de contact 7 et est spécifique de la zone prédéterminée 12a, 12b, 12c, 12d, 12e, 12f en question. La cavité 16 comprend une paroi supérieure 20 horizontale sur laquelle débouchent les canaux 14. La cavité 16 comprend en outre une surface inférieure 22. Dans l'exemple représenté, la surface inférieure 22 de la cavité 16 a une forme de pyramide inversée. La cavité 16 comprend des renforts verticaux (non-représentés). La paroi supérieure 20 et la paroi inférieure 22 de la cavité et les renforts verticaux sont suffisamment rigides pour conférer à la cavité 16 une résistance mécanique à l'écrasement selon la direction verticale V.

La canalisation principale 18 débouche dans la cavité 16, au niveau de la pointe de la pyramide inversée formée par la surface inférieure 22, et de l'autre sur une prise d'air 24a, 24b, 24c, 24d, 24e, 24f située sur une paroi latérale du matelas 4, en vis-à-vis de l'installation de conditionnement d'air 6.

Selon un mode de réalisation, les parois des canaux 14, de la cavité 16 et de la canalisation principale 18 sont faites d'un matériau sensiblement étanche à l'air, à l'humidité et au parfum, par exemple du caoutchouc ou une matière plastique adaptée.

Selon un mode de réalisation, la surface 8 destinée à être en contact avec la personne comprend un matériau perforé, respirant et nettoyable.

Le matelas 4 comprend par exemple six prises d'air latérales 24a, 24b, 24c, 24d, 24e, 24f connectées à six canalisations principales 18 reliées à six cavités 16 communiquant par les canaux 14 avec la surface 8 destinée à être en contact avec les personnes. Chaque prise d'air latérale 24a, 24b, 24c, 24d, 24e, 24f est ainsi en contact pneumatique avec la portion de la surface 8 située dans l'une des zones prédéterminées 12a, 12b, 12c, 12d, 12e, 12f du matelas 4.

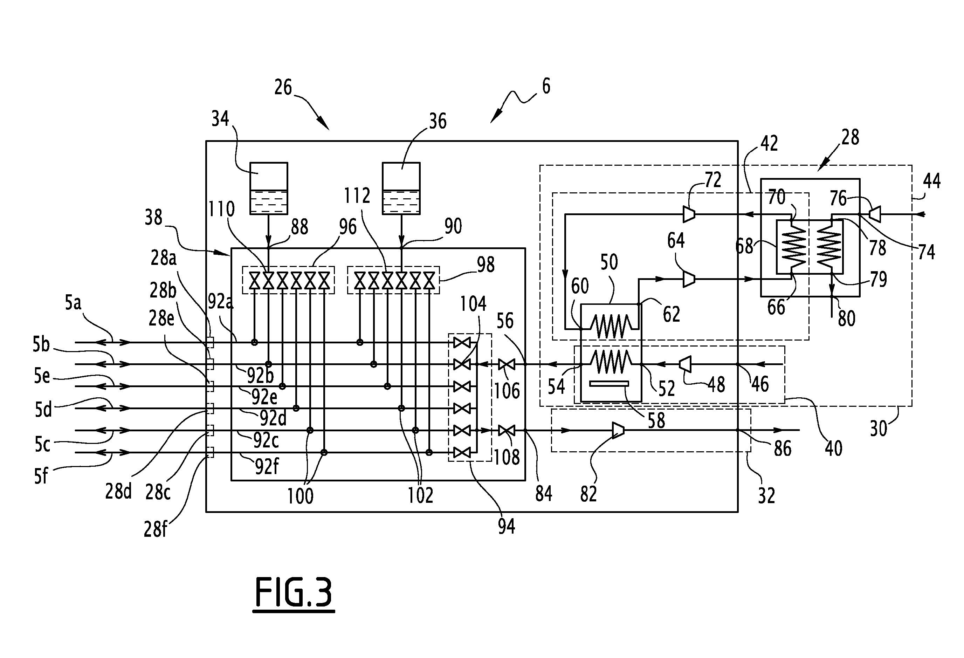

L'installation de conditionnement d'air 6 va maintenant être décrite en référence à la figure 3. L'installation de conditionnement d'air 6 comprend un bloc principal 26 situé à proximité du matelas 4 dans la pièce et un bloc secondaire 28 situé à l'extérieur de la pièce. En variante, l'installation de conditionnement d'air 6 peut se situer hors de la pièce.

Dans l'exemple représenté, le bloc principal 26 se trouve à côté de la cinquième zone prédéterminée 12e du matelas 4. Le bloc principal 26 est connecté par les six tubes de connexion 5a, 5b, 5c, 5d, 5e, 5f aux six prises d'air latérales 24a, 24b, 24c, 24d, 24e, 24f du matelas 4. Les six tubes de connexion 5a, 5b, 5c, 5d, 5e, 5f sont également branchés sur six prises d'air 28a, 28b, 28c, 28d, 28e, 28f située sur une face latérale du bloc principal 26.

L'installation de conditionnement d'air 6 comprend un ensemble de climatisation d'air 30, un système d'extraction d'air 32, une réserve d'eau 34, une réserve de parfum ou d'une substance chimique 36, un sélecteur de voies 38, des canalisations internes et un système d'alimentation électrique et de commande (non-représenté). En variante, la réserve d'eau 34 est remplacée par un raccordement à un réseau de distribution d'eau.

La substance chimique est par exemple un produit anti-acariens.

L'ensemble de climatisation 30 est situé à la fois dans le bloc principal 26, dans le bloc secondaire 28 et entre eux. L'ensemble de climatisation 30 comprend un circuit de chauffage ou de refroidissement 40 de l'air de la pièce, une boucle de réfrigération 42 et une boucle d'évacuation de la chaleur 44.

Le circuit de chauffage ou de réfrigération 40 de l'air de la pièce se situe dans le bloc principal 26. Le circuit de chauffage ou de réfrigération 40 comprend une première entrée d'air 46 située sur une paroi latérale du bloc principal 26, un premier compresseur d'air 48 relié en entrée à la première entrée d'air 46, un premier échangeur de chaleur 50 comportant une entrée d'air 52 reliée à une sortie du premier compresseur d'air 48 et une sortie d'air 54 reliée à une entrée d'air 56 du sélecteur de voies 38. Le premier échangeur de chaleur 50 comprend en outre des résistances électriques 58.

La boucle de réfrigération 42 est un circuit fermé mettant en oeuvre un fluide réfrigérant. La boucle de réfrigération 42 relie le bloc principal 26 au bloc secondaire 28, et inversement. La boucle de réfrigération 42 traverse le premier échangeur 50 qui comporte une entrée 60 et une sortie 62 pour le fluide réfrigérant, celui-ci étant destiné à circuler à contre courant de l'air de la pièce admis dans le premier échangeur 50. La sortie 62 du premier échangeur 50 pour le fluide réfrigérant est connectée à une entrée d'un second compresseur 64 dont une sortie est reliée à une entrée 66 pour fluide réfrigérant d'un second échangeur 68 de chaleur situé dans le bloc secondaire 28. Le second échangeur 68 comporte une sortie 70 pour le fluide réfrigérant connectée à l'entrée d'un organe de détente 72 du fluide réfrigérant, par exemple une vanne de détente. L'organe de détente 72 du fluide réfrigérant comporte une sortie de fluide réfrigérant connectée à l'entrée pour fluide réfrigérant 60 du premier échangeur 50.

La boucle d'évacuation de la chaleur 44 se situe dans le bloc secondaire 28. Elle comprend une seconde entrée d'air 74 située dans une paroi du bloc secondaire 28. L'air destiné à passer par la seconde entrée d'air 74 est de l'air extérieur au bâtiment dans lequel se trouve le bloc principal 26. La seconde entrée d'air 74 est connectée à une entrée d'un troisième compresseur d'air 76, par exemple un ventilateur. Une sortie d'air du troisième compresseur d'air 76 est connectée à une entrée d'air 78 du second échangeur 68, l'air extérieur admis dans le second échangeur 68 étant destiné à circuler à contre-courant du fluide réfrigérant. Le second échangeur 68 comporte une sortie pour l'air extérieur 79 connectée à une sortie d'air 80 située du bloc secondaire.

Le système d'extraction d'air 32 est situé dans le bloc principal 26. Il comprend une pompe d'extraction 82 reliée en entrée à une sortie d'air 84 du sélecteur de voies 38 et en sortie à une sortie d'air 86 située sur une paroi latérale du bloc principal 26.

La réserve d'eau 34 est connectée à une entrée d'eau 88 du sélecteur de voies 38.

La réserve de parfum 36 est connectée à une entrée de parfum 90 du sélecteur de voies 38.

Le sélecteur de voies 38 est situé dans le bloc principal 26. Le sélecteur de voies comprend six lignes d'air 92a, 92b, 92c, 92d, 92e, 92f, un collecteur d'air 94, un collecteur d'eau 96 et un collecteur de parfum 98.

Les lignes d'air 92a, 92b, 92c, 92d, 92e, 92f sont des canalisations comprenant chacune un brumisateur 100 connecté au collecteur d'eau 96 et un brumisateur 102 connecté au collecteur de parfum.

Le collecteur d'air comprend six vannes de réglage 104 connectées aux six lignes d'air 92a, 92b, 92c, 92d, 92e, 92f. Le collecteur d'air 94 est en outre connecté à l'entrée d'air 56 du sélecteur de voies 38 via une vanne d'entrée 106 et à la sortie d'air 84 via une vanne de sortie 108.

Le collecteur d'eau 96 comprend six vannes de réglage 110 de débit connectées aux six lignes d'air 92a, 92b, 92c, 92d, 92e, 92f via les six brumisateurs 100. Le collecteur d'eau 96 est en outre connecté à l'entrée d'eau 88 du sélecteur de voies 38.

Le collecteur de parfum 98 comprend six vannes de réglage 112 de débit connectées aux six lignes d'air 92a, 92b, 92c, 92d, 92e, 92f via les six brumisateurs 102. Le collecteur de parfum 98 est en outre connecté à l'entrée de parfum 90 du sélecteur de voies 38.

Les six lignes d'air 92a, 92b, 92c, 92d, 92e, 92f sont par ailleurs connectées, par leurs extrémités opposées au collecteur d'air 94, aux six prises d'air 28a, 28b, 28c, 28d, 28e, 28f du bloc principal 26 par six canalisations. Les prises d'air 28a, 28b, 28c, 28d, 28e, 28f du bloc principal 26 sont donc des entrées et des sorties d'air pour le sélecteur de voies 38.

Le système d'alimentation électrique et de commande (non-représenté) est connecté aux différents éléments actifs de l'installation de conditionnement d'air 6 : la pompe d'extraction 82, les compresseurs 48, 64, 76, l'organe de détente 72, les résistances électriques 58, les vannes d'entrée 106 et de sortie 108 d'air du sélecteur de voies 38 et les vannes de réglages 104, 110, 112 des lignes d'air 92a, 92b, 92c, 92d, 92e, 92f, des collecteurs d'eau 96 et de parfum 98 du sélecteur de voies 38. Le système d'alimentation électrique et de commande est piloté soit à l'aide d'une interface utilisateur (non-représentée), située par exemple sur une face supérieure du bloc principal 26, soit à l'aide d'une télécommande (non-représentée) du système d'alimentation et de commande.

Le système d'alimentation électrique et de commande comprend aussi des éléments de régulation des températures, des concentrations et des débits des différents flux d'air de l'installation de conditionnement d'air 6.

L'installation de conditionnement d'air 6 est à la fois, sélectivement, un extracteur d'air 38, 32, un appareil de chauffage ou de refroidissement 38, 40, 42, 44 de l'air destiné à traverser la surface 8 destinée à être en contact avec les personnes, un appareil de distribution d'humidité 38, 34, et/ou de parfum 38, 36 dans l'air conditionné.

Le système distributeur ou collecteur d'air 10 situé dans le matelas 4 fonctionne réversiblement, soit comme distributeur d'air, soit comme collecteur d'air, en fonction de l'état de l'installation de conditionnement d'air 6.

Selon un mode particulier de réalisation, le dispositif 10, 6 pour faire circuler le fluide comprend des moyens pour faire circuler l'air de manière pulsée.

Lorsque les personnes couchées dans le lit 1 souhaitent être en mode « extraction d'air », le système d'extraction 38, 32 de l'installation de conditionnement d'air 6 est activé. Pour ce faire, la pompe d'extraction 82 est mise en fonctionnement, la vanne de sortie d'air 108 du sélecteur de voies 38 est ouverte et une ou plusieurs vannes de réglage 104 du collecteur d'air du sélecteur de voies sont sélectivement ouvertes. Ainsi la pompe d'extraction 82 est sélectivement mise en communication pneumatique avec les prises d'air latérales 24a, 24b, 24c, 24d, 24e, 24f du matelas 4, elles-mêmes en communication avec les portions de surface 8 destinée à être en contact avec la personne dans les zones prédéterminées 12a, 12b, 12c, 12d, 12e, 12f du matelas 4. La vanne d'entrée d'air 106 du sélecteur de voie 38 reste fermée dans le mode « extraction d'air ».

Dans chacune des zones prédéterminées 12a, 12b, 12c, 12d, 12e ou 12f concernée par l'activation, de l'air externe au matelas 4 est aspiré dans les canaux 14 de la couche de contact 7, arrive dans la cavité 16, puis passe dans la canalisation principale 18. L'air aspiré est conduit jusqu'à la prise d'air latérale 24a, 24b, 24c, 24d, 24e ou 24f du matelas 4, puis passe dans l'un des tubes de connexion 5a, 5b, 5c, 5d, 5e ou 5f, pour entrer dans l'une des six canalisations conduisant aux lignes d'air 92a, 92b, 92c, 92d, 92e, 92f du sélecteur de voies 38. L'air passe ensuite par la vanne de sortie d'air 108 du sélecteur de voies 38 et par la pompe d'extraction 82, avant d'être rejeté dans la pièce, ou dans une autre pièce par la sortie d'air 84 du bloc principal 26.

La perte de charge étant faible dans les canalisations du sélecteur de voies 38, dans les tubes de connexion 5a, 5b, 5c, 5d, 5e ou 5f et dans le système distributeur ou collecteur 10, le degré d'ouverture des vannes de réglage 104 du collecteur d'air 94 du sélecteur de voie 38 permet de régler sélectivement la pression régnant dans les cavités 16 du matelas 4 et donc le débit d'air entrant dans les canaux 14 dans les zones prédéterminées 12a, 12b, 12c, 12d, 12e, 12f. Lorsque les vannes de réglage 104 sont fermées, le débit est nul. Plus les vannes de réglage 104 sont ouvertes, plus le débit d'air aspiré augmente dans la zone prédéterminée concernée 12a, 12b, 12c, 12d, 12e ou 12f.

Lorsque les personnes souhaitent être en mode « conditionnement d'air », l'ensemble de climatisation 30 de l'installation de conditionnement d'air 6 est activé. Pour ce faire, le circuit de chauffage ou de réfrigération 40 est activé en démarrant le premier compresseur 48 et en ouvrant la vanne d'entrée d'air 106 du sélecteur de voies 38 et une ou plusieurs vannes de réglage 104 du collecteur d'air du sélecteur de voies, en fonction des zones prédéterminées 12a, 12b, 12c, 12d, 12e, 12f du matelas 4 pour lesquelles un conditionnement d'air est souhaité. La vanne de sortie d'air 108 du sélecteur de voies 38 reste fermée dans le mode « conditionnement d'air ».

Si les personnes souhaitent un chauffage de l'air, les résistances électriques 58 sont activées. Si, au contraire, un refroidissement est souhaité, la boucle de réfrigération 42 et la boucle d'évacuation de la chaleur 44 sont activées, par la mise en fonctionnement des second et troisième compresseurs 64 et 76 et de l'organe de détente 72.

Dans le mode « conditionnement d'air », le premier compresseur 48 fait entrer de l'air de la pièce via l'entrée d'air du bloc principal. L'air passe ensuite par le premier échangeur 50 qui, selon le cas, lui cède de la chaleur ou lui en prend. L'air ainsi conditionné passe ensuite, via la vanne d'entrée d'air 106 du sélecteur de voies 38, dans le collecteur d'air 94du sélecteur de voies 38. L'ouverture ou non des vannes de réglage 104 du collecteur d'air 94 permet, comme dans le mode « extraction d'air » de choisir les zones prédéterminées 12a, 12b, 12c, 12d, 12e, 12f du matelas 4 pour lesquelles un conditionnement d'air est souhaité. L'air conditionné suit alors le chemin inverse de celui suivi dans le mode « extraction d'air ». Le degré d'ouverture des vannes de réglage 104 permet de régler le débit d'air conditionné dans les canaux 14 de la couche de contact 7.

Le fonctionnement de la boucle de réfrigération 42 et de la boucle d'évacuation de la chaleur 44 ne sera pas décrit en détail, ces deux boucles étant des éléments classiques d'un climatiseur d'air. Le rôle de la boucle de réfrigération 42, lorsqu'elle est activée, est de transférer la chaleur cédé par l'air de la pièce passant dans le premier échangeur 50 à l'air externe passant dans le second échangeur 68.

En mode « conditionnement d'air », si les personnes souhaitent que l'air conditionné soit humidifié dans une ou plusieurs des zones prédéterminées 12a, 12b, 12c, 12d, 12e ou 12f du matelas 4, une ou plusieurs des vannes de réglage 110 du collecteur d'eau 96 sont ouvertes. Ainsi, de l'eau descend dans les lignes d'air 92a, 92b, 92c, 92d, 92e, 92f correspondantes du sélecteur de voies 38. L'eau est incorporée dans le flux d'air des lignes d'air 92a, 92b, 92c, 92d, 92e, 92f choisies via les brumisateurs 100. L'air injecté dans le matelas 4 est ainsi humidifié.

De même, si la personne souhaite que l'air conditionné soit parfumé dans une ou plusieurs des zones prédéterminées 12a, 12b, 12c, 12d, 12e ou 12f du matelas 4, elle active en outre une ou plusieurs des vannes de réglage 112 du collecteur de parfum 98. Du parfum descend dans les lignes d'air 92a, 92b, 92c, 92d, 92e, 92f correspondantes du sélecteur de voies 38. Le parfum est incorporé dans le flux d'air des lignes d'air 92a, 92b, 92c, 92d, 92e, 92f choisies via les brumisateurs 102. L'air injecté dans le matelas 4 est parfumé.

Si les personnes souhaitent simplement humidifier et/ou parfumer l'air sans le conditionner, les résistances 58 et les boucles de réfrigération et d'évacuation de la chaleur 42 et 44 ne sont pas activées.

Selon un mode particulier de réalisation (non-représenté), les zones prédéterminées 12a, 12b, 12c, 12d, 12e ou 12f sont raccordées indépendamment les une des autres soit à un système de chauffage de l'air, soit à un système de réfrigération de l'air, soit à un système d'extraction d'air. Ainsi, une personne peut choisir des traitements d'air pour les zones prédéterminées 12a, 12c, 12e qui la concernent, tandis que l'autre personne peut choisir des traitements d'air pour les autres zones prédéterminées 12b, 12d, 12f qui la concernent.

Le lit 1 présente l'avantage que l'air est extrait ou conditionné exactement là où se trouvent les personnes, c'est-à-dire au niveau du matelas 4 du lit 1, grâce au dispositif 10, 6 pour faire circuler l'air à travers la surface 8 en contact avec la ou les personnes utilisant le lit 1.

Ainsi, grâce à l'invention, même si les conditions de température, d'humidité ou de concentration en parfum ne sont pas bien contrôlées dans la pièce, ou pas uniformes du fait de courants d'air ou d'une inertie des moyens de conditionnement de l'air de la pièce, la température, l'humidité ou la concentration en parfum ressenties par les personnes allongées dans le lit 1 sont bien contrôlées.

Grâce à l'invention, les problèmes d'inertie, qu'ils concernent la température de l'air autour des personnes, ou bien la concentration en eau ou en parfum, sont réduits, dans la mesure où la quantité d'air à conditionner et l'inertie du dispositif pour faire circuler l'air à travers la surface 8 destinée à être en contact avec les personnes sont réduites.

Ces avantages sont particulièrement appréciables pour une ou des personnes alitées pendant longtemps.

Si un refroidissement est recherché, la chaleur dégagée par la personne elle-même est efficacement compensée en faisant passer de l'air réfrigéré à travers la surface 8 en contact avec la personne.

D'une manière générale, l'énergie consommée pour faire fonctionner l'installation de conditionnement d'air 6, ou les quantités d'eau ou de parfum consommées pour traiter l'air, sont également réduites, car, d'une part, les personnes utilisant le lit 1 sont moins tentées d'augmenter la puissance de l'installation de conditionnement d'air pour vaincre une inertie, et, d'autre part, la quantité d'air à traiter pour obtenir le conditionnement souhaité est réduite.

Selon une variante, le matelas 4 ne comporte pas de cavités 16. Les canaux 14 sont directement connectés aux canalisations principales 18 par une arborescence de canalisations (non-représentées). En variante, les canaux 14 sont directement connectés à deux arborescences de canalisations indépendantes l'une de l'autre, chacune raccordée à l'installation 6, pour permettre la mise en oeuvre de deux types de traitement simultanément, par exemple un soufflage et une extraction.

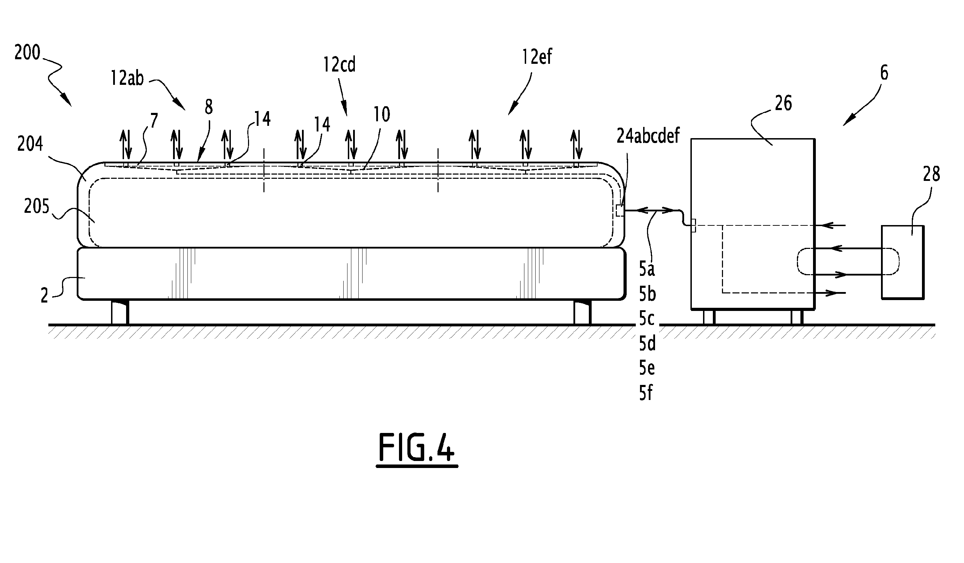

Sur la figure 4 est représenté un lit 200 qui est une variante du lit 1. Les éléments du lit 100 analogues à ceux du lit 1, décrit précédemment, sont repérés par des références identiques et ne sont donc pas décrits à nouveau.

Le lit 200 comprend une housse de matelas 204 qui vient recouvrir un matelas 205 ordinaire. Le système distributeur ou collecteur d'air 10 n'est pas situé dans le matelas 205, mais dans la housse de matelas 204.

Le fonctionnement du lit 200 est analogue à celui du lit 1, une fois que la housse de materas 204 est en place, la housse de matelas du lit 200 jouant le même rôle que le matelas 4 du lit 1 pour faire circuler de l'air à travers la surface 8 destinée à être en contact avec la personne.

Le lit 200 présente les mêmes avantages que le lit 1. Il présente en outre l'avantage que son matelas 205 est un matelas ordinaire. Il est ainsi possible de transformer un lit ordinaire en un lit 200 selon l'invention, en ajoutant la housse de matelas 204 sur le matelas ordinaire 205 et en connectant la housse de matelas 204 à l'installation de conditionnement 6.

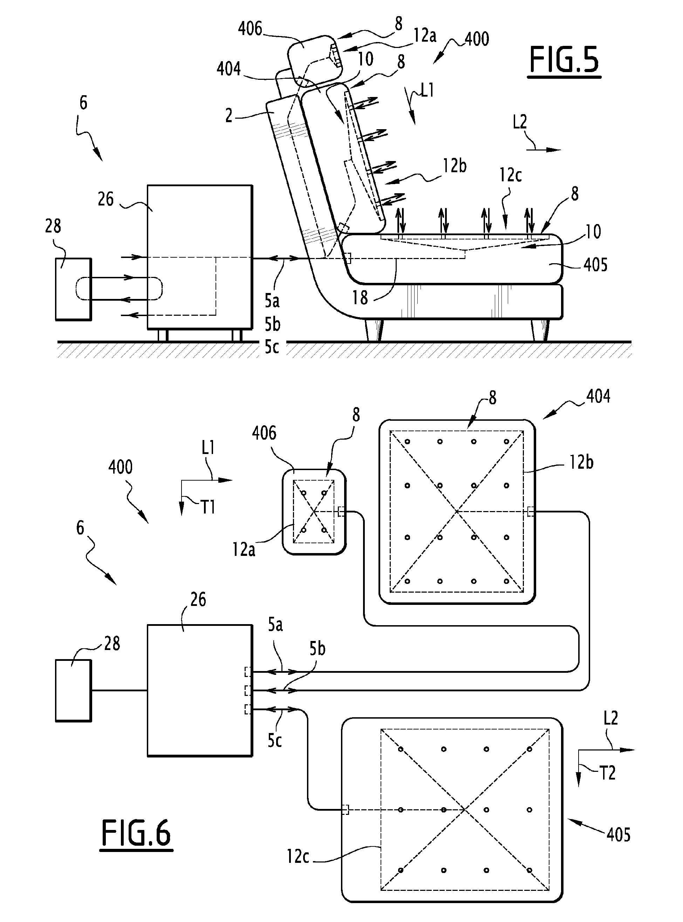

Sur les figures 5 et 6 est représenté un fauteuil 400 pour une personne, qui constitue un second mode de réalisation de l'invention. Les éléments du fauteuil 400 analogues à ceux du lit 1, décrit précédemment, sont repérés par des références identiques et ne sont donc pas décrits à nouveau.

Le fauteuil 400 comprend, non pas un matelas 4, mais un premier coussin 404, un second coussin 405 et un appui-tête 406 connectés à l'installation de conditionnement d'air 6. Les deux coussins 404, 405 et l'appui-tête 406 possèdent chacun une structure et un fonctionnement analogues à ceux du matelas 4 du lit 1.

Le premier coussin 404 et l'appui-tête 406 s'étendent selon une direction L1 et une direction T1 perpendiculaire à la direction L1.

Selon un mode de réalisation particulier, l'appui-tête 406 comprend une zone prédéterminée 12a destinée à se trouver au niveau de la tête d'une personne (non-représentée) assise dans le fauteuil 400.

Le premier coussin 404 comprend une zone prédéterminée 12b destinée à se trouver au niveau du dos de la personne.

Le second coussin 405 s'étend selon une direction L2 et une direction T2 perpendiculaire à la direction L2. Selon un mode de réalisation particulier, le second coussin 405 comprend une zone prédéterminée 12c destinée à se trouver au niveau des jambes de la personne.

Dans l'installation de conditionnement d'air 6, le sélecteur de voies est adapté pour tenir compte du fait que les coussins 404, 405 comprennent trois zones prédéterminées 12a, 12b, 12c au lieu de six, en réduisant à trois le nombre de lignes d'air et de vannes de réglage des collecteurs d'air, d'eau et de parfum du sélecteur de voies.

Le fauteuil 400 présente un fonctionnement et des avantages analogues à ceux du lit 1 et ne seront donc pas décrits.

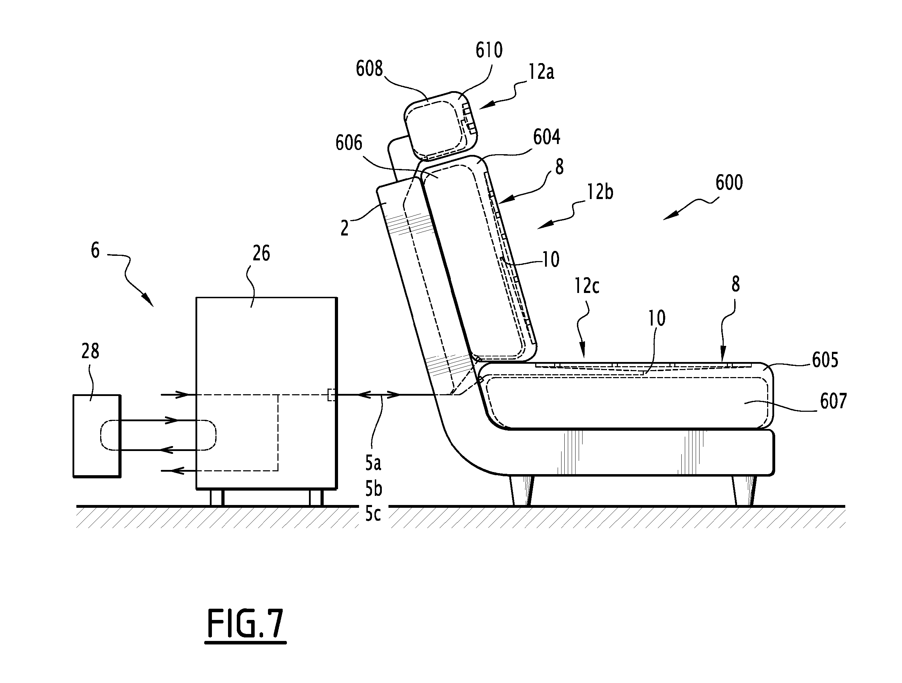

Sur la figure 7 est représenté un fauteuil 600, qui est une variante du fauteuil 400. Les éléments du fauteuil 600 analogues à ceux fauteuil 400, décrit précédemment, sont repérés par des références identiques et ne sont donc pas décrits à nouveau.

Le fauteuil 600 comprend trois housses de coussin 604, 605, 610 qui recouvrent des coussins 606, 607, 608 ordinaires. Le système distributeur ou collecteur d'air 10 n'est pas situé dans les coussins 606, 607, 608, mais dans les housses de coussin 604, 605, 610.

Le fonctionnement du fauteuil 600 est analogue à celui du fauteuil 400, une fois que les housses de coussin 604, 605, 610 sont en place.

Le fauteuil 600 présente les mêmes avantages que fauteuil 200. Il présente en outre l'avantage que ses coussins 606, 607, 608 sont des coussins ordinaires. Il est ainsi possible de transformer un fauteuil ordinaire en un fauteuil 600 selon l'invention, en ajoutant les housses de coussin 604, 605 sur les coussins ordinaires et en connectant les housses de coussin 604, 605, 610 à l'installation de conditionnement d'air 6.

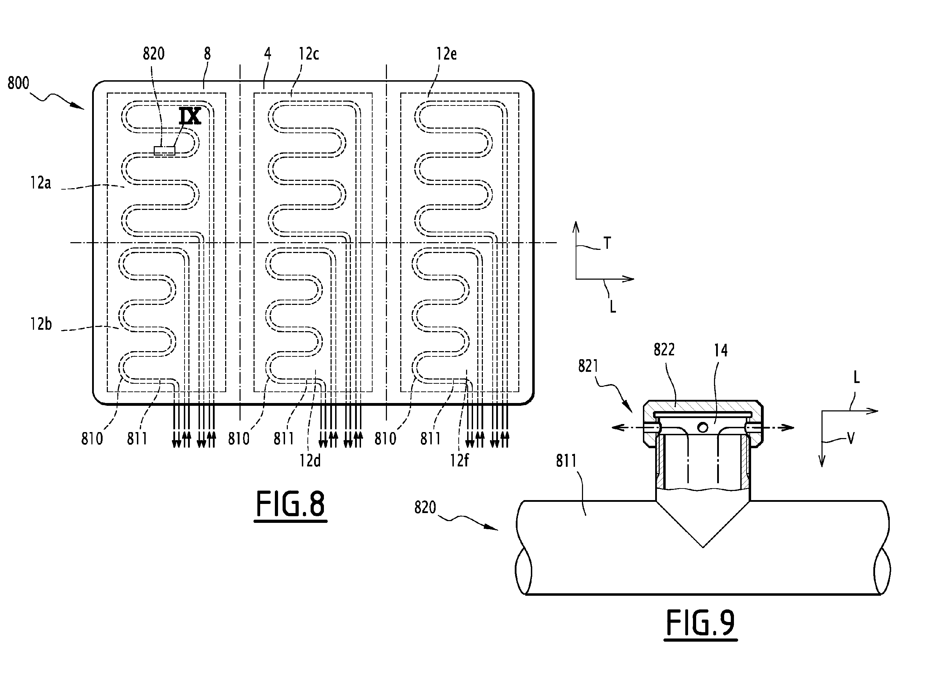

Les figures 8 et 9 illustrent un autre mode de réalisation de l'invention.

Sur la figure 8 est représenté un lit 800 qui est une variante du lit 1 représenté sur les figures 1 et 2. Seuls les éléments qui diffèrent seront décrits ci-après.

Dans chacune des zones prédéterminées 12a, 12b, 12c, 12d, 12e, 12f, le lit 800 comprend deux serpentins 810, 811 parcourant la zone prédéterminée 12a, 12b, 12c, 12d, 12e, 12f pour faire circuler de l'air depuis une entrée située en périphérie du matelas 4, jusqu'à une sortie, également située en périphérie du matelas 4. Les deux serpentins 810, 811 peuvent être deux canaux parallèles ménagés dans un même tube.

Les serpentins 810, 811 sont avantageusement situés dans le matelas 4, sous un sur-matelas poreux.

En variante, les serpentins 810, 811 peuvent être situés dans une housse telle que la housse 204 représentée sur la figure 4, ou entre le matelas 205 et la housse 204.

Les entrées et sorties des serpentins 810, 811 de chaque zone prédéterminée 12b, 12c, 12d, 12e, 12f peuvent par exemple se situer dans les zones prédéterminées 12b, 12d, 12f comme représenté sur la figure 8, ou bien être regroupées à la manière des prises d'air 24a, 24b, 24c, 24d, 24e ou 24f représentées sur la figure 2.

Les entrées des serpentins sont destinées à être connectées à l'installation de conditionnement d'air 6 représentée sur la figure 2. Avec cette installation de conditionnement, ils forment le dispositif pour faire circuler l'air à travers la surface 8 du lit 800.

Les serpentins 810, 811 comprennent une pluralité d'orifices 821. Le rôle des orifices 810, 811 est de laisser passer l'air de l'intérieur des serpentins vers l'extérieur, ou inversement.

Selon un mode particulier de réalisation, les orifices 821 sont réglables, de manière à pouvoir modifier le débit de fluide, avantageusement entre un débit nul (orifice fermé) et un débit maximum prédéterminé.

En utilisation normale, les sorties des serpentins 810, 811 sont fermées.

Un exemple d'orifice réglable 821 du serpentin 811 est représenté sur la figure 9. On voit que l'orifice 821 comprend un capuchon 822 vissé sur une dérivation du serpentin 811. Le fait de visser ou de dévisser le capuchon 822 permet de régler le débit d'air s'échappant ou entrant dans le serpentin 811.

Le réglage des orifices 821 tout au long du serpentin 811 permet de choisir la manière dont l'air est injecté ou aspiré dans la zone prédéterminée 812a, 12d, 12e.

Grâce aux deux serpentins 810, 811, ou plus, il est possible de réaliser les deux types d'opération, injection ou aspiration d'air, simultanément, de façon à réaliser simultanément deux traitements différents, ou plus. Par exemple, il est possible de chauffer la surface 8 du lit 800 et d'extraire de l'air depuis cette surface.

En outre, la forme en serpentins 810, 811 du système distributeur ou collecteur d'air permet un nettoyage facile, par exemple en faisant circuler un produit nettoyant dans les serpentins 810, 811, dont ont aura préalablement fermé les orifices réglables 821 et ouvert les sorties.

Il est possible de programmer le système pour faire varier la température de l'air injecté au cours du temps, par exemple au cours de la nuit. Chacun des serpentins 810, 811, en variante, est agencé en une boucle fermée, avec une entrée raccordée au dispositif de conditionnement d'air. L'air peut circuler le long de la boucle, de telle sorte que la pression est sensiblement uniforme en tout point de la boucle. Les orifices 821 de diffusion ou d'aspiration sont portés par la boucle fermée.

Selon une variante, la couche de soutien est équipée de capteurs de température, l'installation 6 comportant un calculateur renseigné par les capteurs de température et programmé pour réguler la température de la couche de soutien à une valeur de consigne prédéterminée, réglable par l'utilisateur.

## Revendications

1. Elément d'ameublement (1), destiné à recevoir au moins une personne en position assise ou couchée, comprenant une armature (2) et une couche de soutien (4, 204, 205, 404, 405, 406, 604, 605, 606, 607, 608) comportant une surface (8) destinée à être en contact avec ladite personne, **caractérisé en ce qu'**il comprend en outre un dispositif (10, 6) pour faire circuler un fluide à travers ladite surface (8).

2. Elément d'ameublement (1) selon la revendication 1, **caractérisé en ce que** le fluide est de l'air ou de l'air enrichi par exemple en oxygène, ou de l'air purifié.

3. Elément d'ameublement (1) selon la revendication 1 ou 2, **caractérisé en ce que** le dispositif (10, 6) pour faire circuler le fluide à travers ladite surface (8) comprend un système distributeur et collecteur de fluide (10) comportant une pluralité de canaux (14) pour réaliser, sélectivement, une injection du fluide vers l'extérieur de la couche de soutien (4, 204, 205, 404, 405, 406, 604, 605, 606, 607, 608) et/ou une aspiration du fluide vers l'intérieur de la couche de soutien, dans une ou plusieurs zones prédéterminées (12a, 12b, 12c, 12d, 12e, 12f) de ladite couche de soutien (4, 204, 205, 404,405,406,604,605,606,607,608).

4. Elément d'ameublement (1) selon la revendication 3, **caractérisé en ce que** le système distributeur et collecteur de fluide (10) comprend une canalisation principale (18) et, dans chaque zone prédéterminée (12a, 12b, 12c, 12d, 12e, 12f), une ou plusieurs cavités (16) s'étendant dans la couche de soutien (4, 204, 205, 404, 405, 406 604, 605, 606, 607, 608) sous la surface (8) pour distribuer l'air injecté et/ou aspiré.

5. Elément d'ameublement (1) selon l'une quelconque des revendications 3 à 4, **caractérisé en ce que** le système distributeur et collecteur de fluide (10) comprend, dans chaque zone prédéterminée (812a, 12d, 12e), un ou plusieurs serpentins (810, 811) situés dans couche de soutien (4, 204, 205, 404, 405, 604, 605, 606, 607) sous la surface (8), pour faire circuler le fluide de et/ou vers la surface (8), les serpentins comprenant une pluralité d'orifices (821) répartis le long de chaque serpentin (810, 811).

6. Elément d'ameublement (1) selon la revendication 5, **caractérisé en ce que** les orifices (821) sont réglables individuellement entre une position ouverte et une position fermée et **en ce que** le dispositif (10, 6) pour faire circuler le fluide comprend des moyens pour faire circuler un liquide de température contrôlée, sélectivement dans au moins un des serpentins (810, 811).

7. Elément d'ameublement (1) selon l'une quelconque des revendications 3 à 6, **caractérisé en ce qu'**il comprend en outre un ensemble frigorifique (38, 40, 42, 44) pour refroidir, préalablement à son injection, le fluide destiné à être injecté dans une ou plusieurs zones à refroidir prises parmi lesdites une ou plusieurs zones prédéterminées (12a, 12b, 12c, 12d, 12e, 12f).

8. Elément d'ameublement (1) selon l'une quelconque des revendications 3 à 7, **caractérisé en ce qu'**il comprend en outre un ensemble de chauffage (38, 40, 58) pour chauffer, préalablement à son injection, le fluide destiné à être injecté dans une ou plusieurs zones à chauffer prises parmi lesdites une ou plusieurs zones prédéterminées (12a, 12b, 12c, 12d, 12e, 12f).

9. Elément d'ameublement (1) selon l'une quelconque des revendications 3 à 8, **caractérisé en ce qu'**il comprend en outre un appareil de distribution de parfum (38, 36), ou d'une substance chimique, et/ou d'humidité (38, 34), dans le fluide destiné à être injecté dans une ou plusieurs zones à parfumer et/ou à humidifier prises parmi lesdites une ou plusieurs zones prédéterminées (12a, 12b, 12c, 12d, 12e, 12f).

10. Elément d'ameublement (1) selon l'une quelconque des revendications 1 à 9, **caractérisé en ce que** la couche de soutien (4, 204, 205, 404, 405, 406, 604, 605, 606, 607, 608) comprend un coussin ou un matelas et une housse (204, 604, 605, 610), la surface (8) destinée à être en contact avec ladite personne appartenant à ladite housse (204, 604, 605, 610), le système distributeur et collecteur de fluide (10) se situant soit dans la housse (204, 604, 605, 610), soit dans ledit coussin ou matelas, soit entre la housse (204, 604, 605, 610) et ledit coussin ou matelas.

11. Elément d'ameublement (1) selon l'une quelconque des revendications 1 à 10, **caractérisé en ce que** la surface (8) destinée à être en contact avec ladite personne comprend un revêtement perforé, respirant et nettoyable.

12. Procédé de création d'une ambiance autour d'une personne en position assise ou couchée sur un élément d'ameublement (1) selon l'une quelconque des revendications précédentes, **caractérisé en ce qu'**il comporte les étapes suivantes :
- sélection de zones à traiter (12a, 12b, 12c, 12d, 12e, 12f) ;
- pour chaque zone à traiter (12a, 12b, 12c, 12d, 12e, 12f) sélectionnée, choix d'un ou plusieurs traitements pris parmi l'injection de fluide refroidi, l'injection de fluide chauffé, l'injection de fluide humidifié et/ou parfumé et l'aspiration de fluide ;
- pour chaque traitement choisi, sélection d'une intensité du traitement choisi ; et
- à l'aide dudit dispositif (10, 6) pour faire circuler du fluide à travers ladite surface (8), dans chaque zone de traitement sélectionnée (12a, 12b, 12c, 12d, 12e, 12f), injection et/ou aspiration à travers la zone de traitement sélectionnée, selon le traitement choisi, d'un ou plusieurs débits de fluide choisi pour réaliser lesdits traitements choisis avec lesdites intensités sélectionnées.
